# EUROPEAN PATENT SPECIFICATION

(11) **EP 1 677 850 B1**
(45) Date of publication and mention of the grant of the patent: **24.01.2007**
(21) Application number: 04796268.3
(22) Date of filing: 22.10.2004
(51) Int. Cl.: A61L 29/14, A61L 29/16, A61L 29/08

(54) **LUBRICANT COMPOSITIONS, THEIR PREPARATION AND ARTICLES COATED THEREWITH**
GLEITMITTELZUSAMMENSETZUNGEN, IHRE HERSTELLUNG UND DAMIT BESCHICHTETE ARTIKEL
COMPOSITIONS LUBRIFIANTES, LEUR PREPARATION ET ARTICLES ENDUITS DE CES COMPOSITIONS

(30) Priority: 23.10.2003 US 691853
(43) Date of publication of application: 12.07.2006
(73) Proprietor: Sherwood Services AG, 8201 Schaffhausen (CH)
(72) Inventor: MCGHEE, Diane, Hazelwood, MO 63042 (US)
(74) Representative: Tomlinson, Edward James
(86) International application number: PCT/US2004/035249
(87) International publication number: WO 2005/039665

(56) References cited:
- WO-A-00/44414
- WO-A-99/19004
- WO-A-02/058756
- US-A- 5 295 978

## Description

The present invention relates to a lubricious composition suitable for medical devices that may aid medical devices to become slippery when wetted. The lubricious composition of the present invention may be employed to reduce the coefficient of friction of catheters, arterial venous shunts, gastroenteric feed tubes, endotracheal tubes and other medical implants or polymeric substrates. The composition of the present invention may also incorporate pharmacological additive compounds such as anti-microbials that are released in a pharmaceutically acceptable manner. Methods are also provided for the manufacture of the lubricious compositions. Also described are methods for the application of the lubricious compositions with and without pharmaceutical additives to surfaces of medical devices and the resulting coated medical device.

### 2. Background

Known lubricant coatings applied to surfaces of medical devices include coatings of polyvinylpyrrolidone, polyurethane, acrylic polyester, vinyl resin, fluorocarbons, silicone rubber, and combinations of these substances. For example, Micklus et al., U.S. Patent Nos. 4,100,309 and 4,119,094, relate to a hydrophilic coating of polyvinylpyrrolidone-polyurethane interpolymer formed using polyisocyanate. Ratner et al., U.S. Patent No. 3,939,049, relates to a method of grafting hydrogels for lubrication to polymeric substrates using radiation. Hungton et al., U.S. Patent No. 3,975,350, relates to hydrophilic polyurethane polymers for use as lubricants. Storey et al. U.S. Patent No. 3,987,497, relates to a tendon prosthesis having a lubricant hydrogel coating. Many known lubricious coatings are prone to various disadvantages when used in the medical field. Disadvantages of such known lubricants may include insufficiently low coefficient of friction, lack of permanence such as characteristic of silicone or fluorocarbon based coatings, slipperiness when dry as well as wet thus making handling difficult, utilization of hazardous solvents in the manufacture of the same and utilization of unstable reactive materials in the manufacture of the same. Lubricants produced for medical use from unstable reactive material often require the coating solution to be prepared daily or more frequently to be useful and thereby increases waste and expense. Lubricants produced for medical use involving hazardous solvents are undesirable due to patient toxicity concerns and OSHA considerations. Also, lubricant coatings provided for inducing foreign devices into various areas of the body that are susceptible to infection and or thrombogenic reactions have failed to provide a pharmaceutically acceptable carrier for anti-microbial and anti-thrombogenic compounds.

A lubricious composition is needed that when coated upon a medical article and subsequently wetted has sufficient lubricity to be useful in the medical device field such as for medical implants. The composition may also have pharmacological additives such as anti-microbial compounds that can be released in a pharmaceutically acceptable manner. The lubricant coating is desirably capable of adhering to a wide variety of substrates and preferably resist wet abrasion. Further, it would be advantageous to prepare such coating from components that are not health hazards.

### SUMMARY OF THE DISCLOSURE

In an illustrative embodiment, the lubricious composition comprises a hydrophilic polymer, an isocyanate-terminated prepolymer, an alkylester of a carboxylic acid such as ethyl lactate and a solvent selected from tetrahydrofuran (THF), Dimethylformamide (DMF), Methylene chloride, Cyclohexanone, n-methyl pyrrolidone (NMP) and mixtures thereof. It is contemplated that these solvents may be used alone or in combination with each other.

In a further illustrative embodiment, the lubricious composition comprises a hydrophilic polymer, an isocyanate-terminated prepolymer, an alkylester of a carboxylic acid such as ethyl lactate, a solvent selected from tetrahydrofuran (THF), Dimethylformamide (DMF), Methylene chloride, Cyclohexanone, n-methyl pyrrolidone (NMP), mixtures thereof and a pharmaceutical additive.

In yet a further illustrative embodiment, the lubricious composition comprises a hydrophilic polymer, an isocyanate-terminated prepolymer, an alkylester of a carboxylic acid such as ethyl lactate, a solvent selected from tetrahydrofuran (THF), Dimethylformamide (DMF), Methylene chloride, Cyclohexanone, n-methyl pyrrolidone (NMP), mixtures thereof, a pharmaceutical additive and a polymerized urethane. The polymerized urethane increases the binding strength of the inventive coating and controls the rate of release of the pharmaceutical additive. The addition of the polymerized urethane enables the pharmacokinetics of the anti-microbial or other pharmacological additives to be within acceptable pharmaceutical limits.

In a further alternative illustrative embodiment, the lubricious composition comprises a hydrophilic polymer such as polyvinylpyrrolidone, a hexamethylene (HDI) isocyanate-terminated prepolymer, an alkylester of a carboxylic acid and a solvent selected from tetrahydrofuran (THF), Dimethylformamide (DMF), Methylene chloride, Cyclohexanone, n-methyl pyrrolidone (NMP) and mixtures thereof. It is contemplated that this HDI containing lubricious composition can further contain pharmacologic additives and polymerized urethane either alone or in combination.

In another illustrative embodiment, the lubricious composition comprises a hydrophilic polymer such as polyvinylpyrrolidone, an isocyanate-terminated prepolymer, such as ADIPRE^{®} L-100 a TDI - terminated polyether based (PTMEG) prepolymer which is a reaction product of a diisocyanate and a polyalkylene ether glycol, an alkylester of a carboxylic acid such as ethyl lactate and a solvent mixture of tetrahydrofuran (THF) and Cyclohexanone.

A method for making the lubricous compositions of the present disclosure is provided herein. The method comprises blending a solvent selected from the group consisting of tetrahydrofuran, dimethylformamide, methylene chloride, n-methyl pyrrolidone, cyclohexanone and mixtures thereof, a hydrophilic polymer, an isocyanate-terminated prepolymer, and an alkylester of a carboxylic acid. The components are blended together preferably until a uniform solution is formed.

A method for using the lubricous compositions to at least partially coat a surface, preferably the surface of medical devices is provided herein. This process of coating may be carried out by any method known in the art including dip coating or spraying and then air drying or removal of excess lubricant and optionally baking and packaging a medical device either before or after sterilization thereof. The surface may be any surface, preferably a surface of a medical device. The medical device may be optionally cleaned and dried prior to coating.

### DETAILED DESCRIPTION OF THE DISCLOSURE

The lubricious composition of the present invention has been found particularly useful in lowering the coefficient of friction of medical devices such as indwelling thoracic catheters and other medical devices. The lubricious composition is manufactured from a blend of one or more solvents selected from THF, Dimethylformamide (DMF), Methylene chloride, Cyclohexanone, n-methyl pyrrolidone (NMP) and mixtures thereof, an alkylester of a carboxylic acid such as, for example, ethyl lactate, methylbenzoate, or polyacrylate wherein ethyl lactate is preferred, a hydrophilic polymer such as for example polyvinylpyrrolidone, polyvinyl alcohol, polyacrylic acid, copovidone or polyethylene oxide, preferably polyvinylpyrrolidone, to increase hydrophilicity and lubricity, and an isocyanate-terminated prepolymer.

Any isocyanate-terminated prepolymers may be used herein. Preferred isocyanate-terminated prepolymers that can be used according to the disclosure include but are not limited to, polyoxyethylene-based isocyanates such as a toluene or isophorone diisocyanate-based prepolymer such as, for example, Hypol* PreMA G60 (TDI), manufactured by Dow Corporation, Vibrathane^{®} 4,4-diphenylmethane-diisocyanante (MDI) prepolymer, manufactured by Uniroyal, or ADIPRENE^{®} low-free TDI, manufactured by Uniroyal Chemical.

Other isocyanate-terminated prepolymers known in the art may also be used. These preploymers include polytetramethylene ether glycol-diphenylmethane diisocyanate (MDI), polytetramethylene ether glycol-tolylene diisocyanate (TDI), polytetramethylene ether glycol-isophorone diisocyanate, poly(1,4-oxybutylene) glycol-diphenylmethane diisocyanate (MDI), poly(1,4-oxybutylene) glycol-tolylene diisocyanate (TDI), poly(1,4-oxybutylene) glycol-isophorone diisocyanate, polyethylene glycol-diphenylmethane diisocyanate (MDI), polyethylene glycol-tolylene diisocyanate (TDI), polyethylene glycol-isophorone diisocyanate, polypropylene glycol-diphenylmethane diisocyanate (MDI), poly-propylene glycol-tolylene diisocyanate (TDI), polypropylene glycol-isophorone diisocyanate, polycaprolactone-diphenylmethane diisocyanate (MDI), polycaprolactone-tolylene diisocyanate (TDI), polycaprolactone-isophorone diisocyanate, polyethylene adipate-diphenylmethane diisocyanate (MDI), polyethylene adipate-tolylene diisocyanate (TDI), polyethylene adipate-isophorone diisocyanate, polytetra-methylene adipate-diphenylmethane diisocyanate (MDI), polytetramethylene adipate-tolylene diisocyanate (TDI), polytetramethylene adipate-isophorone diisocyanate, polyethylene-propylene adipate-diphenylmethane diisocyanate (MDI), polyethylene-propylene adipate-tolylene, diisocyanate (TDI), or polyethylene-propylene adipate-isophorone diisocyanate polyurethanes.

In an alternative illustrative embodiment, a hexamethylene (HDI) isocyanate-terminated prepolymer can be used. The HDI isocyanate-terminated prepolymers offer improvements in worker safety. The lubricious composition according to the disclosure includes, but is not limited to, ADIPRENE^{®} LFH 710 prepolymer, manufactured by Crompton Corporation (Uniroyal), Middlebury, Connecticut. This HDI prepolymer provides an isocyanate-terminated prepolymer having less than about 0.1% free HDI, which can be beneficial in the management and control of worker exposure to HDI. This prepolymer having relatively low free HDI reduces dermal toxicity that can be associated with other prior art isocyanate-terminated prepolymers.

Polymerized urethanes such as Pellethane^{®}, aromatic ether polyurethane manufactured by Dow Chemical, or Hydrothane^{®} polyurethane, manufactured by CardioTech International, or isocyanate-terminated prepolymers, or both can be used, to enhance binding strength and to adjust release rates of pharmaceutical additives.

The polymerized urethane increases the binding strength of the coating and controls the rate of release and thus enables the pharmacokinetics of the pharmacological additives when utilized to be within acceptable pharmaceutical limits. The durometer of the polymerized urethane used must match the durometer of the medical device to be coated or the functionality of the medical device may become compromised.

Any pharmacological additive may be used. For example anti-microbial additives, such as silver salts or antibiotics, may be uniformly suspended within the lubricous composition. These additives are released on contact with moisture. The rate of release and the lubricious properties of the composition are controlled by altering the ratio of urethane and PVP. For further examples of suitable polyisocyanates see Encyclopedia of Polymer Science and Technology, H. F. Mark, N. G. Gaylord and N. M. Bikeles (eds.) (1969).

Anti-microbial additives utilized as pharmacological additives within the present invention include the biguanides, especially chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampician, bacitracin, neomycin, chloramphenical, miconazole, tolnaftate, quinolones such as oxolinic acid, norfloxacin, nalidix acid, pefloxacin, enoxacin and ciprofloxacin, penicillins such as ampicillin, amoxicillin and piracil, cephalosporins, vancomycin, and combinations of any of the above anti-microbials.

An anti-thrombogenic additive useful as a pharmacological additive according to the present invention would be heparin. Modified forms of heparin may be used to ensure its biological activity and anti-thrombogenic properties. It is contemplated that the urethane component may be modified to more readily accept the heparin or modified heparin molecule. Additionally, organic compounds derived from plants and herbs having desirable pharmacological properties can be utilized as pharmacological additives. Extracts of plants and herbs have been known to possess anti-microbial activity and their use has been shown to be safe for human and animal consumption. Extracts of such plants, known as phytochemicals, may be utilized for their anti-microbial properties. Some of these extracts, such as grapefruit seed extract, Tea Tree Oil and Myrtle Oil and others can be incorporated into the lubricious composition and their anti-microbial properties released to the surrounding tissue in an efficacious manner.

In some illustrative embodiments of the present disclosure colorants, emulsifiers, surfactants, and color stabilizers that are well known within the art may be added to the lubricious composition. The addition of emulsifiers and surfactants may aid in the suspension stability of the lubricous coating vehicle and its surface wettability.

The release rate of pharmacological additives within the lubricious coating formed from the lubricious composition according to the disclosure and the lubricity of the coating may be controlled by the adjustment of the concentration of the PVP.

The lubricious composition of the present disclosure is generally prepared in a dry mixing vat. The lubricious composition is preferably blended at room temperature according to the following component ratios described in weight percent: about 1 to about 4 weight percent, preferably about 1.5 to about 2.5 weight percent of a hydrophilic polymer such as polyvinylpyrrolidone, about 0.5 to about 3 weight percent, preferably about 0.7 to about 1.5 weight percent of isocyanate-terminated prepolymer, about 15 to about 25 weight percent, preferably about 16 to about 23 weight percent alkylester of a carboxylic acid such as ethyl lactate and about 60 to about 90 weight percent, preferably about 70 weight percent to about 80 weight percent of a solvent such as THF.

The components of the lubricious composition of the present disclosure are generally blended at room temperature until the hydrophilic polymer is dissolved within an alkylester of a carboxylic acid such as ethyl lactate and mixed for about 2-4 hours until a uniform solution is formed. Additional solvents of the disclosure, such as THF and Cyclohexanone, are then added to this uniform solution and mixed for an additional about 15-30 minutes until a second uniform solution is formed. Prior to using the composition as a coating, the isocyanate-terminated prepolymer is added to the second uniform solution and mixed for about 30-60 minutes until a final uniform lubricous coating solution is formed.

The coating solution is moisture sensitive and will increase in viscosity if not tightly capped during storage. Prior to coating a surface such as medical devices with the lubricious composition, the particular medical device, such as a catheter, should for best results be cleaned by dip washing in 100 % isopropanol for about 5 seconds and dried by forced air at about 50° to about 90° C to remove surface residual isopropanol and debris. The device is at this point isopropanol free. The medical device is then dip coated for about 5 to 15 seconds in the lubricious composition according to the disclosure, and slowly removed from the solution vat at a rate of about 0.5 to about 1.0 inches per second.

The catheter or other medical device is then air dried at room temperature for about 10 to about 30 seconds to allow any excess lubricious composition to drain off. Optionally, excess lubricant may also be removed using wicking agents known in the art. After air drying, the coated medical devices are optionally but preferably baked in forced air ovens at about 50° to about 90° C +/- 5° for about 30 minutes to about 3 hours, but most preferably for one hour, and then removed from the oven.

Curing temperature and time are dependent upon the isocyanate-terminated prepolymer, solvent selection and concentrations of the components of the lubricious compositions. The coated medical devices are preferably checked for adequate transparency and to ensure that no solvent odor is present.

Preferred methods of making and using lubricious composition of the present disclosure are described in greater detail in the following examples, which are provided for purposes of further illustration.

### Example 1.

A lubricious composition according to the invention was prepared by blending the following components in a mixing vat until dissolved to form a crystal clear to pale yellow solution. The ingredients are blended in order given, in this illustrative example.

| Ingredient | (wt/g) | % |
|---|---|---|
| Ethyl Lactate | 380g | 19.03% |
| PVP K-90 (BASF Kollidion) | 40g | 2.00% |
| Cyclohexanone | 560g | 28.04% |
| THF | 1000g | 50.08% |
| (TDI) ADIPRENE ® L-100 | 17.39g | 0.85% |

The lubricious composition according to the invention was prepared at room temperature. The PVP and ethyl lactate were mixed for about 2-4 hours until the solution was uniform. The Cyclohexanone and THF were added to the uniform solution and mixed for about 15 minutes. The resulting solution was capped to prevent solvent evaporation. One hour prior to using the solution as a coating, the prepolymer ADIPRENE ® L-100 was added to the capped solution and mixed for about 60 minutes until uniform. A medical device was then dipped coated with no dwell as described in Example 3.

### Example 2.

This illustration is a formulation of the present disclosure that includes pharmacological additives. A lubricious composition, containing silver salts for use on PVC medical devices, was prepared by blending the following components in a mixing vat until dissolved to form a crystal clear to pale yellow solution. The release of the silver salt is regulated by Pellethane^{®} to PVP ratio adjustment. The silver salts that can be used are: Giltech Powders 01-07 (this is a water soluble glass silver salt produced by Giltech Ltd.); AlphaSan RC2000 (this is a zirconium/phosphate crystal produced by Milliken Chemical); SSD (silver sulfadiazine manufactured by The Kendall Company in Oriskany Falls, New York); and silver oxide (obtained from Fisher Scientific). Heparin was added in small quantities, 0.5-1.5%, qs. Cyclohexanone.

| Ingredient | (wt/g) | % |
|---|---|---|
| THF | 46.0g | 46.0% |
| Pellethane® AE80 | 3.0g | 3.0% |
| Ethyl Lactate | 19.0g | 19.0% |
| PVP K-90 (BASF Kollidion) | 1.5g | 1.5% |
| Silver Salt (Giltech) | 1.0 g | 1.0% |
| Heparin | 0.5 g | 0.5% |
| Cyclohexanone | 28.0 g | 28.0% |
| TDI (Hypol*PreMA G60) | 1.0g | 1.0% |

The ingredients were mixed at room temperature in two parts comprising a first part of Ethyl Lactate/PVP/Cyclohexanone and a second part of THF/Pellethane. Once both parts had completely dissolved, Ethyl Lactate/PVP/Cyclohexanone solution was added to the THF/Pellethane mixture. In this particular example, it was found that the PVP level should not exceed about 2.5% as higher levels affected coating adherence and silver release. Cyclohexanone was used to qs. solvent level. Prior to coating a TDI (Hypol*PreMA G60) was adding to the above solution and mixed for about 60 minutes. Depending upon the silver complex used, the Cyclohexanone level was adjusted to maximize coating adherence to the PVC device. The amount of silver complex added to the formula is determined by the overall percent of silver loading within the salt complex. Giltech Powders range from 6%-9.5% silver loading. The particle size of silver complex is important in that it may cause problems in coating adherence. That is, larger particle (greater than about 10 microns) size yielded poor coating adherence and uniformity.

### Example 3.

Thoracic catheters made of polyvinyl chloride (PVC) were washed with isopropanol and dried at about 70° C until the isopropanol evaporated off. The catheters were dip coated in the lubricous composition according to Example 1 with no dwell. After dipping and removing at the rate of about one inch per second, the catheters were then immediately baked at about 70° C for about two hours. The resultant coating was transparent and odorless with good bonding. It was observed that when wet the coated catheters were slippery.

The coated catheters were kept at room temperature for 8 months and then tested for relative reduction in Coefficient of Friction. The catheters were tested for coefficient of friction using standard test methods as set forth within ASTM methods for Coefficient of Friction D1894-87. The catheters were lubricious when wet and the friction reduction was 62.4 % as shown below in table 1.

**Table 1 Coefficient of Friction**

| Type | µ=F/W Mean | Percent reduction of (µ) | Stdv. | Min (µ) | Max (µ) | # of Samples |
|---|---|---|---|---|---|---|
| Non Coated Sterile Catheter | 0.543 | NA | 0.012 | 0.508 | 0.582 | 10 |
| Coated Sterile Catheter | 0.204 | 62.43% | 0.004 | 0.197 | 0.214 | 10 |

| | | | | | | |
|---|---|---|---|---|---|---|
| Coefficient of Friction = (µ) | | | | | | |

From the above data, within table 1, it is apparent that the present lubricous composition provided a reduction in the coefficient of friction of 62.4 percent. Accordingly, since it is clear that the catheter was slippery when wet, and the reduction in coefficient of friction was observed, it is clear that the catheter was lubricious.

The lubricious composition prepared in accordance with the present disclosure may be applied as a thin surface film, e.g., less than about 4.0 mil, but most preferably less than about 2.5 mil-in thickness, which upon contact with water or fluid sufficiently reduces the coefficient of friction to aid in the *in vivo* placement of medical devices.

The unexpected significant advantages of the present lubricious composition achieved through the particular composition formulation noted above include decreased wet coefficient of friction, increased adherence with various surfaces, resistance to wet abrasion, arid when pharmaceutical additives were used, the pharmaceutical had efficacious pharmacologic properties.

Medical devices once coated with the lubricious composition of the present disclosure are packaged and sterilized using an appropriate sterilization technique or may be sterilized and then packaged using aseptic technique. Appropriate methods of sterilization and packaging are known to those skilled in the art and include gamma radiation, electronic beam, ethylene oxide, and like methods. Preferably, medical devices coated with the subject lubricious coating are packaged and then sterilized using gamma radiation by cobalt 60 with 1 to 3 mrads, but preferably 2 mrads, in two independent exposure cycles for superior results.

Appropriate packaging for the subject coated medical devices includes metallic foil pouches such as aluminum foil pouches, polyethylene film, ethylene vinyl acetate film, polypropylene film, polyvinyl chloride film, Tyvek^{®} and like packages known to those skilled in the art, but preferably, an aluminum foil cover pouch with an ethylene vinyl acetate film inner liner to prevent moisture absorption by the lubricant. It is contemplated within the scope of the present disclosure that some pharmaceutical additives may be light sensitive and therefore medical devices coated with such additives should be packaged in appropriate light packaging known in the art.

The method of using the subject coated medical devices comprises removing the device from its packaging, applying moisture to the lubricated surface of the device and placing the device as necessary for a particular medical procedure.

It is seen therefore that the present lubricious composition for medical devices provides an effective wet abrasion resistant, low coefficient of friction coating for medical devices and a vehicle for delivering additives such as anti-microbials and other pharmacological active compounds. The lubricious composition, the method of making and using the lubricious composition, the coated surfaces such as medical devices and the method of using the coated medical devices as disclosed and described herein have specific advantages over the heretofore known lubricants for medical devices. The subject lubricious coating vehicle resists wet abrasion, adheres to a variety of surfaces, has a decreased coefficient of friction only when wetted, is biocompatible, and is able to deliver pharmacological active agents with acceptable pharmacokinetic properties. Hence for these reasons, as well as others, it is seen that the present lubricious coating vehicle represents a significant advancement in the art which has substantial commercial significance.

The lubricous composition can be used solely for its lubricous nature. Although the lubricious coating composition described in the illustrative embodiments herein are a series of coatings pertaining to anti-microbial additives and the methods for ensuring that the pharmacokinetics are within efficacious ranges, it should be appreciated that additives within the lubricious coating vehicle could be other desirable pharmaceutical active compounds such as topical anesthetics, anti-inflammatory compounds both non-steroidal and steroidal, spermicidal compounds or the like. Similarly, rather than the traditional pharmaceutical compounds, the additives can be organic compounds with desired pharmacological effects.

## Claims

1. A lubricant composition comprising:
a solvent selected from the group consisting of tetrahydrofuran, dimethylformamide, methylene chloride, n-methyl pyrrolidone, cyclohexanone and mixtures thereof, a hydrophilic polymer, an isocyanate-terminated prepolymer and an alkylester of a carboxylic acid.

2. The composition of claim 1, further comprising a pharmacological additive.

3. The composition of claim 1, wherein said hydrophilic polymer is selected from the group consisting of polyvinylpyrrolidone, polyvinyl alcohol, polyacrylic acid, copovidone and polyethylene oxide.

4. The composition of claim 1, wherein said hydrophilic polymer is polyvinylpyrrolidone.

5. The composition of claim 1, wherein said alkylester of a carboxylic acid is selected from the group consisting of ethyl lactate, methylbenzoate, polyacrylate and a C1-12 alkylester of a carboxylic acid.

6. The composition of claim 2, further comprising a polymerized urethane.

7. The composition of claim 1, wherein said isocyanate-terminated prepolymer is selected from the group consisting of a polyoxyethylene-based isocyanate prepolymer, a toluene diisocyanate-based prepolymer, an isophorone diisocyanate-based prepolymer and a hexamethylene isocyanate-terminated polyether prepolymer.

8. The composition of claim 2 wherein said pharmacological additive is an anti-microbial.

9. The composition of claim 2 wherein said pharmacological additive is selected from the group consisting of chlorhexidine acetate, chlorhexidine gluconate, chlorhexidine hydrochloride, chlorhexidine sulfate, silver acetate, silver benzoate, silver carbonate, silver iodate, silver iodide, silver lactate, silver chloride, silver laurate, silver nitrate, silver oxide, silver palmitate, silver protein, silver sulfadiazine, polymyxin, tetracycline, tobramycin, gentamicin, rifampicin, bacitracin, neomycin, chloramphenicol, oxolinic acid, norfloxacin, nalidix acid, pefloxacin, enoxacin and ciprofloxacin, penicillin, ampicillin, amoxicillin, piracil, cephalosporins, vancomycin and mixtures thereof.

10. A method for producing a lubricant composition comprising the steps of:
blending a solvent selected from the group consisting of tetrahydrofuran, dimethylformamide, methylene chloride, n-methyl pyrrolidone, cyclohexanone and mixtures thereof, a hydrophilic polymer, an isocyanate-terminated prepolymer, and an alkylester of a carboxylic acid.

11. The method of claim 10 wherein the blending is carried out until an uniform solution is formed.

12. The method of claim 10 further comprising adding a pharmacological additive.

13. The method of claim 10, wherein said hydrophilic polymer is selected from the group consisting of polyvinylpyrrolidone, polyvinyl alcohol, polyacrylic acid, copovidone and polyethylene oxide.

14. The method of claim 10, wherein said hydrophilic polymer is polyvinylpyrrolidone.

15. The method of claim 10, wherein said alkylester of a carboxylic acid is selected from the group consisting of ethyl lactate, methylbenzoate,polyacrylate and a C1-12 alkylester of a carboxylic acid.

16. The method of claim 12, further comprising adding a polymerized urethane.

17. The method of claim 16, wherein the ratio of said polymerized urethane to hydrophilic polymer is adjusted to achieve appropriate pharmacokinetics release rate of said pharmacologic additive.

18. The method of claim 10, wherein said isocyanate-terminated prepolymer is selected from the group consisting of a polyoxyethylene-based isocyanate prepolymer, a toluene diisocyanate-based prepolymer, an isophorone diisocyanate-based prepolymer and a hexamethylene isocyanate-terminated polyether prepolymer.

19. The method of claim 12, wherein said pharmacological additive is an anti-microbial.

20. The method of claim 12 wherein said pharmacological additive is selected from the group consisting of chlorhexidine acetate, chlorhexidine gluconate, chlorhexidine hydrochloride, chlorhexidine sulfate, silver acetate, silver benzoate, silver carbonate, silver iodate, silver iodide, silver lactate, silver chloride, silver laurate, silver nitrate, silver oxide, silver palmitate, silver protein, silver sulfadiazine, polymyxin, tetracycline, tobramycin, gentamicin, rifampicin, bacitracin, neomycin, chloramphenicol, oxolinic acid, norfloxacin, nalidix acid, pefloxacin, enoxacin and ciprofloxacin, penicillin, ampicillin, amoxicillin, piracil, cephalosporins, vancomycin and mixtures thereof.

21. A method of coating at least a portion of a surface with a lubricious composition comprising the steps of:
applying a lubricious composition to at least a portion of a surface, said lubricious composition comprising a solvent selected from the group consisting of tetrahydrofuran, dimethylformamide, methylene chloride, n-methyl pyrrolidone, cyclohexanone and mixtures thereof, a hydrophilic polymer, an isocyanate-terminated prepolymer and an alkylester of a carboxylic acid.

22. The method of coating according to claim 21 further comprising curing the applied lubricious composition.

23. The method of coating according to claim 21 wherein said surface is washed and dried prior to applying said lubricious composition.

24. The method of coating according to claim 21 wherein said surface is a surface of a medical device.

25. The method according to claim 21, wherein said lubricous composition further comprises a pharmacological agent.

26. The method according to claim 24, wherein said medical device is selected from the group consisting of a catheter, an arterial venous shunt, a gastroenteric feed tube and an endotracheal tube.

27. The method according to claim 24, wherein said medical device is an urological catheter formed of polyvinyl chloride.

28. A medical device at least partially coated with a lubricious composition comprising:
a solvent selected from the group consisting of tetrahydrofuran, dimethylformamide, methylene chloride, n-methyl pyrrolidone, cyclohexanone and mixtures thereof, a hydrophilic polymer, an isocyanate-terminated prepolymer and an alkylester of a carboxylic acid.

29. The medical device according to claim 28 wherein the lubricious composition is further cured.

30. The medical device according to claim 28, wherein said lubricous composition further comprises a pharmacological agent.

31. The medical device according to claim 28, wherein said medical device is selected from the group consisting of a catheter, an arterial venous shunt, a gastroenteric feed tube and an endotracheal tube.

32. The medical device according to claim 28, wherein said medical device is an urological catheter formed of polyvinyl chloride.

33. The medical device according to claim 28 wherein said solvent is a mixture of tetrahydrofuran and cyclohexanone, the hydrophilic polymer is polyvinylpyrrolidone, the isocyanate-terminated prepolymer is TDI - terminated polyether based (PTMEG) prepolymer which is the reaction product of a diisocyanate and a polyalkylene ether glycol and the alkylester of a carboxylic acid is ethyl lactate.

34. The lubricant composition according to claim 1 wherein the solvent is a mixture of tetrahydrofuran and cyclohexanone, the hydrophilic polymer is polyvinylpyrrolidone, the isocyanate-terminated prepolymer is a TDI - terminated polyether based (PTMEG) prepolymer which is the reaction product of a diisocyanate and a polyalkylene ether glycol, and the alkylester of a carboxylic acid is ethyl lactate.

35. The method according to claim 10 wherein the solvent is a mixture of tetrahydrofuran and cyclohexanone, the hydrophilic polymer is polyvinylpyrrolidone, the isocyanate-terminated prepolymer is a TDI - terminated polyether based (PTMEG) prepolymer which is the reaction product of a diisocyanate and a polyalkylene ether glycol, and the alkylester of carboxylic acid is ethyl lactate.

## Patentansprüche

1. Gleitmittelzusammensetzung, umfassend:
ein Lösungsmittel, ausgewählt aus der Gruppe, bestehend aus Tetrahydrofuran, Dimethylformamid, Methylenchlorid, N-Methylpyrrolidon, Cyclohexanon und Gemischen davon, ein hydrophiles Polymer, ein Isocyanat-terminiertes Präpolymer und einen Alkylester einer Carbonsäure.

2. Zusammensetzung nach Anspruch 1, weiterhin umfassend ein pharmakologisches Additiv.

3. Zusammensetzung nach Anspruch 1, wobei das hydrophile Polymer ausgewählt ist aus der Gruppe, bestehend aus Polyvinylpyrrolidon, Polyvinylalkohol, Polyacrylsäure, Copovidon und Polyethylenoxid.

4. Zusammensetzung nach Anspruch 1, wobei das hydrophile Polymer Polyvinylpyrrolidon ist.

5. Zusammensetzung nach Anspruch 1, wobei der Alkylester einer Carbonsäure ausgewählt ist aus der Gruppe, bestehend aus Ethyllactat, Methylbenzoat, Polyacrylat und einem C1-12 Alkylester einer Carbonsäure.

6. Zusammensetzung nach Anspruch 2, weiterhin umfassend ein polymerisiertes Urethan.

7. Zusammensetzung nach Anspruch 1, wobei das Isocyanat-terminierte Präpolymer ausgewählt ist aus der Gruppe, bestehend aus einem Isocyanat-Präpolymer auf Basis von Polyoxyethylen, einem Präpolymer auf Basis von Toluoldiisocyanat, einem Präpolymer auf Basis von Isophorondiisocyanat und einem Hexamethylenisocyanat-terminierten Polyether-Präpolymer.

8. Zusammensetzung nach Anspruch 2, wobei das pharmakologische Additiv eine antimikrobielle Substanz ist.

9. Zusammensetzung nach Anspruch 2, wobei das pharmakologische Additiv ausgewählt ist aus der Gruppe, bestehend aus Chlorhexidinacetat, Chlorhexidingluconat, Chlorhexidin-Hydrochlorid, Chlorhexidinsulfat, Silberacetat, Silberbenzoat, Silbercarbonat, Silberjodat, Silberjodid, Silberlactat, Silberchlorid, Silberlaurat, Silbernitrat, Silberoxid, Silberpalmitat, Silberprotein, Silbersulfadiazin, Polymyxin, Tetracyclin, Tobramycin, Gentamicin, Rifampicin, Bacitracin, Neomycin, Chloramphenicol, Oxolinsäure, Norfloxacin, Nalidixinsäure, Pefloxacin, Enoxacin, und Ciprofloxacin, Penicillin, Ampicillin, Amoxicillin, Piracil, Cephalosporinen, Vancomycin und Gemischen davon.

10. Verfahren zur Herstellung einer Gleitmittelzusammensetzung, umfassend die Schritte:
Mischen eines Lösungsmittels, ausgewählt aus der Gruppe, bestehend aus Tetrahydrofuran, Dimethylformamid, Methylenchlorid, N-Methylpyrrolidon, Cyclohexanon und Gemischen davon, eines hydrophilen Polymers, eines Isocyanat-terminierten Präpolymers und eines Alkylesters einer Carbonsäure.

11. Verfahren nach Anspruch 10, wobei das Mischen ausgeführt wird, bis eine gleichförmige Lösung gebildet wird.

12. Verfahren nach Anspruch 10, weiterhin umfassend das Zugeben eines pharmakologischen Additivs.

13. Verfahren nach Anspruch 10, wobei das hydrophile Polymer ausgewählt wird aus der Gruppe, bestehend aus Polyvinylpyrrolidon, Polyvinylalkohol, Polyacrylsäure, Copovidon und Polyethylenoxid.

14. Verfahren nach Anspruch 10, wobei das hydrophile Polymer Polyvinylpyrrolidon ist.

15. Verfahren nach Anspruch 10, wobei der Alkylester einer Carbonsäure ausgewählt wird aus der Gruppe, bestehend aus Ethyllactat, Methylbenzoat, Polyacrylat und einem C1-12 Alkylester einer Carbonsäure.

16. Verfahren nach Anspruch 12, weiterhin umfassend die Zugabe eines polymerisierten Urethans.

17. Verfahren nach Anspruch 16, wobei das Verhältnis des polymerisierten Urethans zu dem hydrophilen Polymer eingestellt wird um eine geeignete pharmakokinetische Freisetzungsgeschwindigkeit des pharmakologischen Additivs zu erreichen.

18. Verfahren nach Anspruch 10, wobei das Isocyanat-terminierte Präpolymer ausgewählt wird aus der Gruppe, bestehend aus einem lsocyanat-Präpolymer auf Basis von Polyoxyethylen, einem Präpolymer auf Basis von Toluoldiisocyanat, einem Präpolymer auf Basis von Isophorondiisocyanat und einem Hexamethylenisocyanat-terminierten Polyether-Präpolymer.

19. Verfahren nach Anspruch 12, wobei das pharmakologische Additiv eine antimikrobielle Substanz ist.

20. Verfahren nach Anspruch 12, wobei das pharmakologische Additiv ausgewählt wird aus der Gruppe, bestehend aus Chlorhexidinacetat, Chlorhexidingluconat, Chlorhexidin-Hydrochlorid, Chlorhexidinsulfat, Silberacetat, Silberbenzoat, Silbercarbonat, Silberjodat, Silberjodid, Silberlactat, Silberchlorid, Silberlaurat, Silbernitrat, Silberoxid, Silberpalmitat, Silberprotein, Silbersulfadiazin, Polymyxin, Tetracyclin, Tobramycin, Gentamicin, Rifampicin, Bacitracin, Neomycin, Chloramphenicol, Oxolinsäure, Norfloxacin, Nalidixinsäure, Pefloxacin, Enoxacin, und Ciprofloxacin, Penicillin, Ampicillin, Amoxicillin, Piracil, Cephalosporinen, Vancomycin und Gemischen davon.

21. Verfahren zum Beschichten mindestens eines Abschnitts einer Oberfläche mit einer Gleitmittelzusammensetzung, umfassend die Schritte:
Aufbringen einer Gleitmittelzusammensetzung auf mindestens einen Abschnitt einer Oberfläche, wobei die Gleitmittelzusammensetzung ein Lösungsmittel, ausgewählt aus der Gruppe, bestehend aus Tetrahydrofuran, Dimethylformamid, Methylenchlorid, N-Methylpyrrolidon, Cyclohexanon und Gemischen davon, ein hydrophiles Polymer, ein Isocyanat-terminiertes Präpolymer und einen Alkylester einer Carbonsäure umfasst.

22. Beschichtungsverfahren nach Anspruch 21, weiterhin umfassend das Härten der aufgebrachten Gleitmittelzusammensetzung.

23. Beschichtungsverfahren nach Anspruch 21, wobei die Oberfläche vor dem Aufbringen der Gleitmittelzusammensetzung gewaschen und getrocknet wird.

24. Beschichtungsverfahren nach Anspruch 21, wobei die Oberfläche eine Oberfläche einer medizinischen Vorrichtung ist.

25. Beschichtungsverfahren nach Anspruch 21, wobei die Gleitmittelzusammensetzung weiterhin einen pharmakologischen Wirkstoff umfasst.

26. Beschichtungsverfahren nach Anspruch 24, wobei die medizinische Vorrichtung ausgewählt wird aus der Gruppe, bestehend aus einem Katheter, einem Arterie-Vene-Shunt, einem Magen-Darm-Zufuhrschlauch und einem endotrachealen Schlauch.

27. Verfahren nach Anspruch 24, wobei die medizinische Vorrichtung ein aus Polyvinylchlorid gebildeter urologischer Katheter ist.

28. Medizinische Vorrichtung, welche mindestens teilweise beschichtet ist mit einer Gleitmittelzusammensetzung, umfassend:
ein Lösungsmittel, ausgewählt aus der Gruppe, bestehend aus Tetrahydrofuran, Dimethylformamid, Methylenchlorid, N-Methylpyrrolidon, Cyclohexanon und Gemischen davon, ein hydrophiles Polymer, ein Isocyanat-terminiertes Präpolymer und einen Alkylester einer Carbonsäure.

29. Medizinische Vorrichtung nach Anspruch 28, wobei die Gleitmittelzusammensetzung weiterhin gehärtet ist.

30. Medizinische Vorrichtung nach Anspruch 28, wobei die Gleitmittelzusammensetzung weiterhin einen pharmakologischen Wirkstoff umfasst.

31. Medizinische Vorrichtung nach Anspruch 28, wobei die medizinische Vorrichtung ausgewählt ist aus der Gruppe, bestehend aus einem Katheter, einem Arterie-Vene-Shunt, einem Magen-Darm-Zufuhrschlauch und einem endotrachealen Schlauch.

32. Medizinische Vorrichtung nach Anspruch 28, wobei die medizinische Vorrichtung ein aus Polyvinylchlorid gebildeter urologischer Katheter ist.

33. Medizinische Vorrichtung nach Anspruch 28, wobei das Lösungsmittel ein Gemisch aus Tetrahydrofuran und Cyclohexanon ist, das hydrophile Polymer Polyvinylpyrrolidon ist, das lsocyanat-terminierte Präpolymer ein Präpolymer auf Basis von TDI-terminiertem Polyether (PTMEG) ist, welches das Reaktionsprodukt eines Diisocyanats und eines Polyalkylenetherglycols ist, und der Alkylester einer Carbonsäure Ethyllactat ist.

34. Gleitmittelzusammensetzung nach Anspruch 1, wobei das Lösungsmittel ein Gemisch aus Tetrahydrofuran und Cyclohexanon ist, das hydrophile Polymer Polyvinylpyrrolidon ist, das Isocyanat-terminierte Präpolymer ein Präpolymer auf Basis von TDI-terminiertem Polyether (PTMEG) ist, welches das Reaktionsprodukt eines Diisocyanats und eines Polyalkylenetherglycols ist, und der Alkylester einer Carbonsäure Ethyllactat ist.

35. Verfahren nach Anspruch 10, wobei das Lösungsmittel ein Gemisch aus Tetrahydrofuran und Cyclohexanon ist, das hydrophile Polymer Polyvinylpyrrolidon ist, das Isocyanat-terminierte Präpolymer ein Präpolymer auf Basis von TDI-terminiertem Polyether (PTMEG) ist, welches das Reaktionsprodukt eines Diisocyanats und eines Polyalkylenetherglycols ist, und der Alkylester einer Carbonsäure Ethyllactat ist.

## Revendications

1. Composition lubrifiante comprenant :
un solvant choisi dans le groupe comprenant le tétrahydrofuranne, le diméthylformamide, le chlorure de méthylène, le n-méthyl pyrrolidone, le cyclohexanone et les mélanges de ceux-ci, un polymère hydrophile, un prépolymère terminé par un isocyanate et un alkylester d'un acide carboxylique.

2. composition selon la revendication 1, comprenant en outre un additif pharmacologique.

3. Composition selon la revendication 1, dans laquelle ledit polymère hydrophile est choisi dans le groupe comprenant le polyvinylpyrrolidone, l'alcool polyvinylique, l'acide polyacrylique, le copovidone et l'oxyde de polyéthylène.

4. Composition selon la revendication 1, dans laquelle ledit polymère hydrophile est le polyvinylpyrrolidone.

5. Composition selon la revendication 1, dans laquelle ledit alkylester d'un acide carboxylique est choisi dans le groupe comprenant le lactate d'éthyle, le méthylbenzoate, le polyacrylate et un alkylester en C1 à C12 d'un acide carboxylique.

6. Composition selon la revendication 2, comprenant en outre un uréthane polymérisé.

7. Composition selon la revendication 1, dans laquelle ledit prépolymère terminé par un isocyanate est choisi dans le groupe comprenant un prépolymère d'isocyanate à base de polyoxyéthylène, un prépolymère à base de toluène diisocyanate, un prépolymère à base d'isophorone diisocyanate et un prépolymère de polyéther terminé par un hexaméthylène isocyanate.

8. Composition selon la revendication 2 dans laquelle ledit additif pharmacologique est un agent antimicrobien.

9. Composition selon la revendication 2 dans lequel ledit additif pharmacologique est choisi dans le groupe comprenant l'acétate de chlorhexidine, le gluconate de chlorhexidine, l'hydrochlorure de chlorhexidine, le sulfate de chlorhexidine, l'acétate d'argent, le benzoate d'argent, le carbonate d'argent, l'iodate d'argent, l'iodure d'argent, le lactate d'argent, le chlorure d'argent, le laurate d'argent, le nitrate d'argent, l'oxyde d'argent, le palmitate d'argent, la protéine d'argent, le sulfadiazine d'argent, la polymyxine, la tétracycline, la tobramycine, la gentamicine, la rifampicine, la bacitracine, la néomycine, la chloramphénicol, l'acide oxolinique, la norfloxacine, l'acide nalidixique, la pefloxacine, l'énoxacine et la ciprofloxacin, la pénicilline, l'ampicilline, l'amoxicilline, le piracil, les céphalosporines, la vancomycine et les mélanges de ceux-ci.

10. Procédé de production d'une composition lubrifiante comprenant les étapes suivantes :
le mélange d'un solvant choisi dans le groupe comprenant la tétrahydrofuranne, le diméthylformamide, le chlorure de méthylène, le n-méthyl pyrrolidone, le cyclohexanone et les mélanges de ceux-ci, un polymère hydrophile, un prépolymère terminé par un isocyanate et un alkylester d'un acide carboxylique.

11. Procédé selon la revendication 10 dans lequel le mélange est réalisé jusqu'à ce qu'une solution soit formée.

12. Procédé selon la revendication 10 comprenant en outre l'ajout d'un additif pharmacologique.

13. Procédé selon la revendication 10, dans lequel ledit polymère hydrophile est choisi dans le groupe comprenant le polyvinylpyrrolidone, l'alcool polyvinylique, l'acide polyacrylique, le copovidone et l'oxyde de polyéthylène.

14. Procédé selon la revendication 10, dans lequel ledit polymère hydrophile est le polyvinylpyrrolidone.

15. Procédé selon la revendication 10, dans lequel ledit alkylester d'un acide carboxylique est choisi dans le groupe comprenant le lactate d'éthyle, le méthylbenzoate, le polyacrylate et un alkylester en C1-C12 d'un acide carboxylique.

16. Procédé selon la revendication 12, comprenant en outre l'ajout d'un uréthane polymérisé.

17. Procédé selon la revendication 16, dans lequel le rapport dudit uréthane polymérisé par rapport au polymère hydrophile est ajusté de manière à obtenir un taux de libération pharmacocinétique approprié dudit additif pharmacologique.

18. Procédé selon la revendication 10, dans lequel ledit prépolymère terminé par un isocyanate est choisi dans le groupe comprenant un prépolymère d'isocyanate à base de polyoxyéthylène, un prépolymère à base de toluène diisocyanate, un prépolymère à base d'isophorone diisocyanate et un prépolymère de polyéther terminé par l'hexaméthylène isocyanate.

19. Procédé selon la revendication 12, dans lequel ledit additif pharmacologique est un agent antimicrobien.

20. Procédé selon la revendication 12 dans lequel ledit additif pharmacologique est choisi dans le groupe comprenant l'acétate de chlorhexidine, le gluconate de chlorhexidine, le chlorhydrate de chlorhexidine, le sulfate de chlorhexidine, l'acétate d'argent, le benzoate d'argent, le carbonate d'argent, l'iodate d'argent, l'iodure d'argent, le lactate d'argent, le chlorure d'argent, le laurate d'argent, le nitrate d'argent, l'oxyde d'argent, le palmitate d'argent, la protéine d'argent, la sulfadiazine d'argent, la polymyxine, la tétracycline, la tobramycine, la gentamicine, la rifampicine, la bacitracine, la néomycine, le chloramphénicol, l'acide oxolinique, la norfloxacine, l'acide nalidixique, la pefloxacine, l'énoxacine et la ciprofloxacin, la pénicilline, l'ampicilline, l'amoxicilline, la piracil, les céphalosporines, la vancomycine et les mélanges de ceux-ci.

21. Procédé de revêtement d'au moins une portion d'une surface avec une composition lubrifiante comprenant les étapes suivantes :
l'application d'une composition lubrifiante à au moins une portion d'une surface de ladite composition lubrifiante comprenant un solvant choisi dans le groupe comprenant la tétrahydrofuranne, le diméthylformamide, le chlorure de méthylène, le n-méthyl pyrrolidone, le cyclohexanone et les mélanges de ceux-ci, un polymère hydrophile, un prépolymère terminé par un isocyanate et un alkylester d'un acide carboxylique.

22. Procédé de revêtement selon la revendication 21 comprenant en outre la réticulation de la composition lubrifiante appliquée.

23. Procédé de revêtement selon la revendication 21 dans lequel ladite surface est lavée et séchée avant l'application de ladite composition lubrifiante.

24. Procédé de revêtement selon la revendication 21 dans lequel ladite surface est une surface d'un dispositif médical.

25. Procédé selon la revendication 21, dans lequel ladite composition lubrifiante comprend en outre un agent pharmacologique.

26. Procédé selon la revendication 24, dans lequel ledit dispositif médical est choisi dans le groupe comprenant un cathéter, une dérivation artérielle, un tube d'alimentation gastro-entérique et un tube endotrachéal.

27. Procédé selon la revendication 24, dans lequel ledit dispositif médical est un cathéter urologique formé de chlorure de polyvinyle.

28. Dispositif médical au moins partiellement enduit d'une composition lubrifiante comprenant :
un solvant choisi dans le groupe comprenant la tétrahydrofuranne, le diméthylformamide, le chlorure de méthylène, le n-méthyle pyrrolidone, le cyclohexanone, et les mélanges de ceux-ci, un polymère hydrophile, un prépolymère terminé par un isocyanate et un alkylester d'un acide carboxylique.

29. Dispositif médical selon la revendication 28 dans lequel la composition lubrifiante est à nouveau réticulée.

30. Dispositif médical selon la revendication 28, dans lequel ladite composition lubrifiante comprend en outre un agent pharmacologique.

31. Dispositif médical selon la revendication 28, dans lequel ledit dispositif médical est choisi dans le groupe comprenant un cathéter, une dérivation artérielle, un tube d'alimentation gastro-entérique et un tube endotrachéal.

32. Dispositif médical selon la revendication 28, dans lequel ledit dispositif médical est un cathéter urologique formé de chlorure de polyvinyle.

33. Dispositif médical selon la revendication 28 dans lequel ledit solvant est un mélange de tétrahydrofuranne et de cyclohexanone, le polymère hydrophile est le polyvinylpyrrolidone, le prépolymère terminé par un isocyanate est un prépolymère à base de polyéther terminé par TDI qui est le produit de réaction d'un diisocyanate et d'un éther glycol de polyalkylène et l'alkylester d'un acide carboxylique est le lactate d'éthyle.

34. Composition lubrifiante selon la revendication 1 dans laquelle le solvant est un mélange de tétrahydrofuranne et de cyclohexanone, le polymère hydrophile est le polyvinylpyrrolidone, le prépolymère terminé par un isocyanate est un prépolymère à base de polyéther terminé par TDI qui est le produit de réaction d'un diisocyanate et d'un éther glycol de polyalkylène et l'alkylester d'un acide carboxylique est le lactate d'éthyle.

35. Procédé selon la revendication 10 dans lequel le solvant est un mélange de tétrahydrofuranne et de cyclohexanone, le polymère hydrophile est le polyvinylpyrrolidone, le prépolymère terminé par un isocyanate est un prépolymère à base de polyéther terminé par TDI qui est le produit de réaction d'un diisocyanate et d'un éther glycol de polyalkylène et l'alkylester d'un acide carboxylique est le lactate d'éthyle.
